# EUROPEAN PATENT APPLICATION

(11) **EP 4 692 798 A1**
(43) Date of publication of application: **11.02.2026**
(21) Application number: 25192843.8
(22) Date of filing: 30.07.2025
(51) Int. Cl.: G01N 33/543, G01N 33/545

(54) **TEST REAGENT INCLUDING THIOUREA COMPOUND AND RESIN PARTICLES EACH HAVING SULFUR ELEMENT, AND METHOD OF DETECTING TARGET SUBSTANCE IN SPECIMEN BY IN VITRO DIAGNOSIS USING TEST REAGENT**

(30) Priority: 06.08.2024 JP 2024130088
(71) Applicant: Canon Kabushiki Kaisha, Tokyo, 146-8501 (JP)
(72) Inventor: UNNO, Tomohiro, Tokyo (JP); DOI, Noriyuki, Tokyo (JP); NATORI, Ryo, Tokyo (JP); TAKAHATA, Nozomu, Tokyo (JP); KOSAKI, Yusuke, Tokyo (JP)
(74) Representative: WESER & Kollegen Patentanwälte PartmbB

(57) **Abstract**

Provided are a test reagent that is reduced in nonspecific adsorption and is capable of detecting a low-concentration target substance, and a detection method including using the test reagent. The test reagent is a test reagent for in vitro diagnosis including: a thiourea compound; and resin particles, wherein the resin particles each have a sulfur element.

## Description

### TECHNICAL FIELD

The present disclosure relates to a test reagent including a thiourea compound and resin particles each having a sulfur element, and a detection method.

### BACKGROUND

In in vitro diagnostics for use in diagnosis of diseases, a method utilizing resin particles has been known as a method of detecting a target substance. This method includes causing a target substance and a ligand to react with each other by fixing the ligand to each of resin particles and bringing the resultant into contact with a sample.

In the above-mentioned method, during the contact between the resin particles and the sample, the target substance and impurities in the sample each nonspecifically adsorb to the surface of each of the resin particles, but not the ligand, and the adsorption becomes noise in measurement in some cases. Accordingly, in such method utilizing the resin particles as described above, it is desired that the resin particles each have a low adsorbing property to a substance except the target substance, which is called nonspecific adsorption.

There is a method of coating the surface of each of the resin particles with a biologically derived macromolecule, such as albumin, casein, or gelatin, in a downstream process as a method of reducing the nonspecific adsorption. However, those biologically derived substances have different physical properties for each production lot in some cases.

Thus, when the surface of each of the resin particles is coated in the downstream process, a method using a hydrophilic macromolecule having high biocompatibility instead of the biologically derived macromolecule is also effective as the method of reducing the nonspecific adsorption. However, when the adsorption of the macromolecules to the resin particles is based on physical adsorption, the macromolecules may be liberated by dilution, and the nonspecific adsorption cannot be sufficiently suppressed in some cases.

A technology including introducing a sulfur element into the vicinity of the surface of each of resin particles each having polystyrene as a main chain has been known as a method by which the nonspecific adsorption of the surface of the resin particle can be prevented. It is conceivable that the nonspecific adsorption can be prevented by: an improvement in hydrophilicity of each of the resin particles through the incorporation of the sulfur element; and electrostatic repulsion between the particles. In each of International Publication No. WO2012/133771 and Japanese Patent Laid-Open No. 2014-153140, there is a disclosure of a reagent using resin particles each having, on its surface, a side chain including a sulfonic acid group or a sulfinyl group.

The related art has been susceptible to improvement in terms of nonspecific adsorption and the detection of a low-concentration target substance.

### SUMMARY

The present disclosure relates to a test reagent for in vitro diagnosis including a thiourea compound; and resin particles, wherein the resin particles each have a sulfur element.

The present disclosure also relates to a method of detecting a target substance in a specimen by in vitro diagnosis, the method including mixing the above-mentioned test reagent and a specimen that may contain a target substance.

According to the present disclosure, there can be provided the test reagent for in vitro diagnosis, which significantly reduces nonspecific adsorption and can detect a low-concentration target substance. Further, there can be provided the detection method including using the test reagent.

Features of the present disclosure will become apparent from the following description of embodiments. The following description of embodiments is described by way of example.

### DESCRIPTION OF THE EMBODIMENTS

Embodiments of the present disclosure are described in detail below, but the technical scope of the present disclosure is not limited to these embodiments. The expression "(meth)acrylic" as used herein means "acrylic or methacrylic." For example, the expression "(meth)acrylate" means "an acrylate or a methacrylate."

A test reagent according to the present disclosure is a test reagent for in vitro diagnosis including: a thiourea compound; and resin particles, wherein the resin particles each have a sulfur element. The inventors of the present invention have investigated a latex agglutination method serving as one method of detecting a target substance. As a result, in the case of related-art sulfur element-containing resin particles, the following so-called false positive was observed in a system for measuring the amount of a low-concentration target substance in a specimen: even a negative specimen was judged to be positive owing to the occurrence of nonspecific adsorption. The inventors have revealed that the suppression of nonspecific adsorption particularly in a sulfur element-containing side chain portion in each of the resin particles is required.

Specifically, the inventors of the present invention have investigated the latex agglutination method serving as one method of detecting a target substance. As a result, in the case of such sulfur element-containing resin particles as described in, for example, International Publication No. WO2012/133771 or Japanese Patent Laid-Open No. 2014-153140, the following so-called false positive was observed in a system for measuring the amount of a low-concentration target substance in a specimen: even a negative specimen was judged to be positive owing to the occurrence of nonspecific adsorption. The inventors of the present invention have made extensive investigations to find a solution method from the mechanism via which a specific causative substance in the specimen nonspecifically adsorbs to each of the resin particles. As a result, the inventors have arrived at the invention of a test reagent, which is reduced in nonspecific adsorption and can detect even a low-concentration target substance, through use of a test reagent simultaneously including a thiourea compound and sulfur element-containing resin particles.

That is, the present disclosure is directed to provide a test reagent including a thiourea compound and resin particles each having a sulfur element, the test reagent being reduced in nonspecific adsorption and capable of detecting a low-concentration target substance, and a detection method including using the above-mentioned test reagent.

### <Resin Particles>

The resin particles of the present disclosure are each characterized by having a sulfur element. The presence of the sulfur element may contribute to the suppression of nonspecific adsorption between the resin particles and the detection of a low-concentration target substance. A possible reason therefor is as described below. When the resin particles each include a sulfur element-containing structure, each of the particles has some degree of polarity and obtains moderate hydrophilicity. Thus, while the dispersibility of the resin particles is improved, their agglutination at the time of the detection of the target substance is hardly inhibited. Accordingly, the presence of the sulfur element may contribute to the suppression of the nonspecific adsorption between the resin particles and the detection of the low-concentration target substance.

Although the sulfur element-containing structure is not particularly limited, the resin particles each preferably have a polymer having at least one of a sulfide group and a thiol group from the viewpoint of the moderate hydrophilicity described above. The presence of a sulfide group or a thiol group more easily suppresses the inhibition of the agglutination of the particles at the time of the detection of a target substance due to the presence of excessive hydrophilicity. Any other sulfur element-containing structure is, for example, a sulfo group or a thioketone group.

The amount of the sulfur element in the resin particles in the present disclosure determined with an X-ray photoelectron spectrometer (ESCA) is preferably 0.1 atm% or more and 8.0 atm% or less, more preferably 0.5 atm% or more and 6.0 atm% or less. As described in detail later, the resin particles each preferably have a polymer having a carboxy group or a carboxy group-derived structure. In this case, the amount of the sulfur element in the resin particles determined with the ESCA is still more preferably 1.5 atm% or more and 2.0 atm% or less from the viewpoint of detecting a low-concentration target substance. When the resin particles are each free of a carboxy group or a carboxy group-derived structure, the amount of the sulfur element determined with the ESCA is still more preferably 3.1 atm% or more and 6.0 atm% or less from the viewpoint of suppressing nonspecific adsorption.

It is conceivable that when the resin particles each contain a certain amount of a sulfur element, the resin particles have dispersibility enough to suppress nonspecific adsorption therebetween and the inhibition of their agglutination at the time of the detection of a target substance can be suppressed, and hence an excellent effect is obtained. As described in detail later, with regard to nonspecific adsorption between a substance in a specific specimen and the sulfur element-containing resin particles, a suppressing effect on the nonspecific adsorption may be easily obtained when the particles are mixed with a thiourea compound in a test reagent. For the control of the amount of the sulfur element in the resin particles determined with the ESCA within the most preferred range, for example, in the case where a sulfide group is incorporated as a sulfur element-containing structure into each of the resin particles, a method for the control is, for example, substitution of part of the sulfide groups with an amine group.

To the extent that the object of the present disclosure can be achieved, the chemical structure of each of the resin particles is not particularly limited except that the structure contains a sulfur element. However, the particles each preferably have a polymer having, in its main chain, a structure derived from at least one of styrenes and (meth)acrylates. In addition, the particles each preferably have a polymer having a hydroxy group. Further, the particles each preferably have a polymer having at least one of a carboxy group and a carboxy group-derived structure.

The resin particles each preferably have at least one of a structure derived from the styrenes and a structure derived from the (meth)acrylates because each of the resin particles has a high glass transition temperature and hence has sufficient strength. Further, the styrenes are preferred because of the following reason: since the styrenes each have high hydrophobicity and hence easily agglutinate at the time of the detection of a target substance in a specimen, a detection sensitivity-improving effect is obtained. Examples of the styrenes and the (meth)acrylates that may be used in this embodiment are listed below, but the styrenes and the (meth)acrylates are not limited thereto. In addition, two or more kinds of oily radically polymerizable monomers may be used.

Examples of the styrenes include styrene, α-methylstyrene, β-methylstyrene, o-methylstyrene, m-methylstyrene, p-methylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, and p-phenylstyrene.

Examples of the (meth)acrylates include: glycidyl (meth)acrylate, methyl acrylate, ethyl acrylate, n-propyl acrylate, iso-propyl acrylate, n-butyl acrylate, iso-butyl acrylate, tert-butyl acrylate, n-amyl acrylate, n-hexyl acrylate, 2-ethylhexyl acrylate, n-octyl acrylate, n-nonyl acrylate, cyclohexyl acrylate, benzyl acrylate, dimethyl phosphate ethyl acrylate, diethyl phosphate ethyl acrylate, dibutyl phosphate ethyl acrylate, and 2-benzoyloxyethyl acrylate; and methyl methacrylate, ethyl methacrylate, n-propyl methacrylate, iso-propyl methacrylate, n-butyl methacrylate, iso-butyl methacrylate, tert-butyl methacrylate, n-amyl methacrylate, n-hexyl methacrylate, 2-ethylhexyl methacrylate, n-octyl methacrylate, n-nonyl methacrylate, diethyl phosphate ethyl methacrylate, and dibutyl phosphate ethyl methacrylate.

A possible reason why the resin particles each preferably have a hydroxy group is as described below. For example, the structure derived from the styrenes given as an example of the structure that the polymer of each of the resin particles preferably has in its main chain has high hydrophobicity. Probably because of the foregoing, the presence of a hydroxy group that is hydrophilic can suppress the nonspecific agglutination of the resin particles due to a hydrophobic interaction therebetween. A biologically derived hydrophilic additive such as BSA has heretofore been used for suppressing the agglutination. However, concern is raised about variation in performance between the lots of the additive and nonspecific adsorption to a substance in a specimen, the adsorption being peculiar to BSA. In contrast, when the resin particles each have a hydroxy group, nonspecific adsorption between the resin particles can be suppressed without use of an additive such as BSA. Accordingly, such design that a false positive and a false negative hardly occur is easily performed.

A possible reason why the resin particles each preferably have a polymer having a carboxy group or a carboxy group-derived structure is as described below. For example, in a process of causing a ligand, such as an antibody or an antigen, to adsorb to each of the resin particles, a carboxy group may serve as a reactive functional group to facilitate the achievement of both of high reactivity and the suppression of interparticle agglutination due to charge repulsion between the resin particles. Accordingly, the ligand, such as an antibody or an antigen, uniformly adsorbs to each of the resin particles with ease, and hence an improving effect on sensitivity to a low-concentration substance is obtained. Examples of the other reactive functional group include a maleimide group, an amide group, a tosyl group, an amino group, an epoxy group, and a thiol group. The carboxy group-derived structure that the polymer of each of the resin particles has refers to a structure having bonded thereto the ligand, such as an antigen or an antibody, through a carboxy group. That is, when the resin particles each have the ligand, such as an antigen or an antibody, the antigen or the antibody can be bonded to the resin particle through a carboxy group.

One method for producing a polymer having a hydroxy group or a carboxy group in accordance with target design is, for example, as follows: glycidyl (meth)acrylate is incorporated into each of the resin particles; and its epoxy moiety is opened and bonded to form a polymer of target design. For example, when mercaptopropanediol or aminopropanediol is caused to react with glycidyl (meth)acrylate, a structure having a hydroxy group is obtained. In addition, similarly, for example, when mercaptosuccinic acid is caused to react with glycidyl (meth)acrylate, a structure having a carboxy group is obtained.

Further, a structure derived from a group of radically polymerizable monomers each having crosslinkability may be incorporated into each of the resin particles each having a sulfur element. Examples of the group of radically polymerizable monomers each having crosslinkability include diethylene glycol diacrylate, triethylene glycol diacrylate, tetraethylene glycol diacrylate, polyethylene glycol diacrylate, 1,6-hexanediol diacrylate, neopentyl glycol diacrylate, tripropylene glycol diacrylate, polypropylene glycol diacrylate, 2,2'-bis(4-(acryloxydiethoxy)phenyl)propane, trimethylolpropane triacrylate, tetramethylolmethane tetraacrylate, ethylene glycol dimethacrylate, diethylene glycol dimethacrylate, triethylene glycol dimethacrylate, tetraethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2'-bis(4-(methacryloxydiethoxy)phenyl)propane, 2,2'-bis(4-(methacryloxypolyethoxy)phenyl)propane, trimethylolpropane trimethacrylate, tetramethylolmethane tetramethacrylate, divinylbenzene, divinylnaphthalene, and divinyl ether. However, the group of radically polymerizable monomers is not limited thereto to the extent that the object of the present disclosure can be achieved. In addition, two or more kinds of the groups of radically polymerizable monomers each having crosslinkability may be used in combination.

A radical polymerization initiator may be used at the time of the production of the resin particles. Examples of the polymerization initiator include 4,4'-azobis(4-cyanovaleric acid), 2,2'-azobis[2-methyl-N-(2-hydroxyethyl)propionamide], 2,2'-azobis[N-(2-carboxyethyl)-2-methylpropionamidine] tetrahydrate, 2,2'-azobis(2-methylpropionamidine) dihydrochloride, 2,2'-azobis[2-(2-imidazolin-2-yl)propane], 2,2'-azobis[2-(2-imidazolin-2-yl)propane] dihydrochloride, 2,2'-azobis[2-(2-imidazolin-2-yl)propane] disulfate dihydrate, potassium peroxodisulfate, ammonium peroxodisulfate, and sodium peroxodisulfate. However, the polymerization initiator is not limited thereto to the extent that the object of the present disclosure can be achieved.

The particle diameter of each of the resin particles is preferably 0.05 µm or more and 1.00 µm or less, more preferably 0.10 µm or more and 0.60 µm or less, still more preferably 0.10 µm or more and 0.40 µm or less in terms of number-average particle diameter. When the particle diameter is 0.10 µm or more and 0.40 µm or less, centrifugal separation for purification can be performed when the resin particles are used by being dispersed in an aqueous medium. Accordingly, handling is facilitated, and particle sedimentation hardly occurs during storage in a state of a resin particle dispersion.

### <Ligand>

The ligand in the present disclosure refers to be a compound that is specifically bonded to the receptor of a specific target substance. The site at which the ligand is bonded to the target substance is predetermined, and the ligand has a selectively or specifically high affinity for the target substance. Although the ligand is not particularly limited, an antigen or an antibody is preferably used. Examples of the other ligand include: an enzyme protein and a substrate thereof; a signal substance, such as a hormone or a neurotransmitter, and a receptor thereof; and a nucleic acid.

### <Ligand-bonded Resin Particles>

The test reagent according to the present disclosure may include, as its resin particles, resin particles before ligand bonding, or may include ligand-bonded resin particles. The ligand-bonded resin particles are each a particle in which the above-mentioned ligand and the reactive functional group of the resin particle before the ligand bonding are bonded to each other. That is, the resin particles in the present disclosure may be particles each having a ligand (e.g., an antigen or an antibody) bonded to its surface (reactive functional group). A mode of bonding between the reactive functional group and the ligand is not particularly limited, but the reactive functional group is preferably selected from a carboxy group, an amino group, a thiol group, and a maleimide group in terms of selective bonding property to the ligand. Further, a carboxy group is most preferably selected in terms of ease in reaction. As a method for a chemical reaction by which the carboxy group and the ligand are chemically bonded to each other, a hitherto known method may be applied to the extent that the object of the present disclosure can be achieved. For example, a carbodiimide-mediated reaction or an NHS ester activation reaction is a frequently used chemical reaction method, but the method for the chemical reaction by which the carboxy group and the ligand are chemically bonded to each other is not limited thereto.

### <Activated Resin Particles>

In a ligand-bonded support in which a ligand is bonded, when the shape of a base material is a particle, the specific surface area of the surface of the support is maximized, and hence its effect can be most suitably exhibited. The ligand-bonded resin particle in which the shape of the base material is a particle is a resin particle having a selectively or specifically high affinity (activated) for a target substance, and is hence also referred to as "activated resin particle." The ligand-bonded support in which the shape of the base material is a particle, that is, the activated resin particle can be extremely preferably applied as a method of detecting a target substance in a specimen in in vitro diagnosis, and an example thereof may be application as a particle for a latex agglutination method. When general particles are used as particles for a latex agglutination method, an antigen (antibody) serving as the target substance, foreign matter in serum or plasma, or the like nonspecifically adsorbs to the surface of each of the particles, and hence concern is raised in that unintended interparticle agglutination occurs owing to the adsorption to impair the accuracy of an immunological test. When the activated resin particles in the present disclosure are used, unintended interparticle agglutination due to nonspecific adsorption can be reduced, and a low-concentration target substance can be detected.

### <Test Reagent for Use in Detection of Target Substance in Specimen by In Vitro Diagnosis>

A test reagent for use in the detection of a target substance in a specimen by in vitro diagnosis according to the present disclosure is characterized by including a thiourea compound and resin particles each having a sulfur element. Thus, a low-concentration target substance can be detected while nonspecific adsorption is suppressed. A possible reason for the foregoing is as described below. The thiourea compound can take a form having a proper amount of a thiol group through an equilibrium reaction, and hence traps a substance in a specific specimen that is liable to nonspecifically adsorb to each of the resin particles each having a sulfur element. Accordingly, the combination of the thiourea compound and the resin particles each having a sulfur element may suppress the occurrence of nonspecific adsorption between the substance in the specimen and the resin particles, and that between the resin particles, and may enable the detection of the low-concentration target substance. Further, as described above, the control of the amount of the sulfur element in the resin particles may be able to achieve both the suppression of the nonspecific adsorption and the detection of the low-concentration target substance at more excellent levels.

The inventors of the present invention have conceived that it is important to use the thiourea compound to cause a proper amount of a thiol to be present in the test reagent. When the amount is not excessively large, the reactivity of an antibody, an antigen, or the like in the test reagent is sufficiently held. When the amount is not insufficient, a sufficient amount of the substance in the specific specimen that is liable to nonspecifically adsorb to each of the resin particles can be trapped. For example, even when a compound actively supplying a thiol group such as 2-mercaptoethanol known as a reducing agent, or a compound free of a form having a thiol group such as urea, which served as a chaotropic agent as in the thiourea compound, was added to the test reagent, an effect in the present disclosure was not sufficiently obtained.

On the assumption that the test reagent is aqueous and in consideration of solubility in water, the thiourea compound preferably contains a compound represented by the following formula (1).

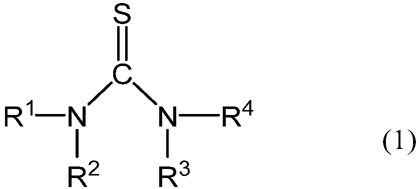

In the formula (1), R¹ to R⁴ each independently represent a hydrogen atom, an alkyl group having 1 to 4 carbon atoms that may be branched, an allyl group, an acetyl group, or a group represented by the following formula (2).

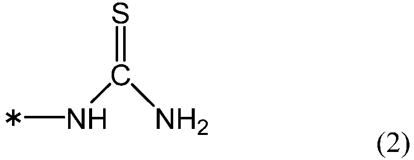

In the formula (2), * represents a bonding position in the compound represented by the formula (1).

Examples of the compound represented by the formula (1) include thiourea, 1,3-diethyl-2-thiourea, 2,5-dithiobiurea, 1-methyl-2-thiourea, 1-ethyl-2-thiourea, 1-allyl-2-thiourea, 1-tert-butyl-2-thiourea, N,N'-dimethylthiourea, 1,3-dibutyl-2-thiourea, 1,3-di-tert-butyl-2-thiourea, 1-acetylthiourea, and trimethylthiourea, and thiourea is more preferred.

From the viewpoint of suppressing nonspecific adsorption, the content of the thiourea compound in the test reagent is preferably 0.0005 M or more and 2 M or less, more preferably 0.005 M or more and 2 M or less. The term "test reagent" as used herein refers to the whole amount of the test reagent. As described later, for example, when the test reagent is formed of two liquids including a buffer solution and a resin particle dispersion, the term refers to the total amount of the buffer solution and the resin particle dispersion (the whole amount of the test reagent), and hence the content of the thiourea compound in the test reagent refers to a content with respect to the whole amount of the test reagent.

The test reagent according to the present disclosure may be a test reagent including two or more liquids including a buffer solution and a resin particle dispersion. For example, in the case where the buffer solution is set as a first reagent (specimen diluent) and the resin particle dispersion is set as a second reagent, the resin particles in the present disclosure may be incorporated into the second reagent (resin particle dispersion). In this case, the first reagent (buffer solution) may be free of the resin particles and contain the thiourea compound.

When the content of the thiourea compound in the test reagent according to the present disclosure is represented by W(A), and the content of the sulfur element-containing resin particles in the test reagent is represented by W(B), the mass ratio W(A)/W(B) may be 0.2 or more and 450 or less. In addition, the amount of the activated resin particles to be incorporated into the test reagent according to the present disclosure is preferably from 0.001 mass% to 20 mass%, more preferably from 0.01 mass% to 10 mass%.

The pH of the buffer solution in the present disclosure is preferably 6.0 or more and 9.0 or less, more preferably 6.5 or more and 8.5 or less. When the pH is set within the above-mentioned ranges, a thiol group can be caused to be present without excess or deficiency in the test reagent by the equilibrium reaction of the thiourea compound. Although an agent for adjusting the pH is not particularly limited, an acid serving as such agent is, for example, hydrochloric acid, acetic acid, or oxalic acid, and a base serving as such agent is, for example, sodium hydroxide, potassium hydroxide, sodium carbonate, or potassium carbonate.

In addition, the test reagent according to the present disclosure may include a third substance in addition to the above-mentioned resin particles and the like to the extent that the object of the present disclosure can be achieved.

The test reagent according to the present disclosure preferably includes a buffer. Although the kind of the buffer is not particularly limited, at least one kind of buffer selected from the group consisting of: MES; Bis-Tris; ADA; PIPES; ACES; BES; MOPSO; MOPS; TES; HEPES; TAPSO; POPSO; HEPSO; EPPS; Tricine; Bicine; TAPS; CHES; and CAPS is preferably used from the viewpoint that a pH for causing the thiourea compound to have a proper amount of a thiol group through an equilibrium reaction is easily held. Examples of the other buffer agent include PBS, such Tris hydrochloric acid as typified by Tris-HCl, boric acid, phosphoric acid, acetic acid, citric acid, succinic acid, phthalic acid, glutaric acid, maleic acid, glycine, and salts thereof.

In addition, the test reagent according to the present disclosure preferably includes a chelating agent. The chelating agent adsorbs, for example, a metal ion such as a calcium ion in the test reagent. It is conceivable that as a result of the foregoing, the thiourea compound is suppressed from adsorbing to the metal ion, and hence easily adsorbs a nonspecific causative substance in a specimen in a more selective manner. Examples of the chelating agent include EDTA, EDTA-2Na, EDTA-3Na, EDTA-4Na, EDTA-2K, EDTA-3K, EDTA-4K, edetate calcium disodium, and edetate calcium dipotassium.

In addition, a water-soluble additive, for example, a water-soluble polymer, such as polyethylene glycol, carboxymethyl cellulose, methyl cellulose, dextran, polyvinylpyrrolidone, polyglycosylethyl methacrylate, pullulan, dextran, elsinan, or polyacrylic acid, may be incorporated thereinto in order to increase the sensitivity of the reaction. Further, for example, a protein, such as casein or gelatin, or a decomposition product or modified product thereof, a quaternary ammonium salt such as choline chloride, a polyanion, a chaotropic ion (e.g., Cl⁻, I⁻, or SCN⁻), an amino acid, a surfactant, and a sugar may be incorporated thereinto for the purposes of, for example, an improvement in specificity and an improvement in stability of the test reagent.

### <Detection Method>

A method of detecting a target substance in a specimen by in vitro diagnosis according to the present disclosure is characterized by including a step of mixing the test reagent according to the present disclosure and a specimen that may contain a target substance (step of obtaining a reaction liquid). When the test reagent is a test reagent including two or more liquids including a buffer solution and a resin particle dispersion, the above-mentioned step of obtaining the reaction liquid may include the steps of: mixing the buffer solution and the specimen to provide a mixed liquid; and mixing the mixed liquid and the resin particle dispersion. At this time, the buffer solution may be free of the resin particles and contain the thiourea compound, and the resin particle dispersion may contain the resin particles.

The content of the thiourea compound in the above-mentioned reaction liquid may be set to 0.0005 M or more and 2 M or less. The activated resin particles and the specimen are preferably mixed at a pH in the range of from 3.0 to 11.0. In addition, a mixing temperature falls within the range of from 20°C to 50°C, and a mixing time falls within the range of from 1 minute to 20 minutes. In addition, in this detection method, a solvent is preferably used. In addition, the concentration of the activated resin particles in the detection method according to the present disclosure is preferably from 0.001 mass% to 5 mass%, more preferably from 0.01 mass% to 1 mass% in a reaction system. The detection method according to the present disclosure is characterized by optically detecting interparticle agglutination caused as a result of the mixing of the resin particles in the present disclosure and the specimen. By optically detecting the interparticle agglutination, the target substance in the specimen is detected, and the concentration of the target substance can be measured. In a method of optically detecting the agglutination reaction, an optical instrument that can detect a scattered light intensity, a transmitted light intensity, an absorbance, and the like only needs to be used to measure the variations of these values.

### <Measurement of Molecular Weight by GPC>

The molecular weight of the polymer (resin particles) in the present disclosure having at least one of a structure derived from at least one of styrenes and (meth)acrylates, a structure having a hydroxy group, or a structure having a carboxy group may be measured by GPC. A method therefor is as described below.

Methanol for high-performance liquid chromatography and a sample are placed in a vial and the sample is dissolved. After the dissolution of the sample has been recognized, filtration is performed with a disposable disk filter manufactured by Tosoh Corporation (product name: Myshori Disk, aperture: 0.5 µm), and a substance passed through the filter is adopted as a GPC sample.

A sample solution is adjusted so that its concentration may be about 1.0 mass%.

Measurement is performed with the sample solution under the following conditions.

| | |
|---|---|
| ·Apparatus: | Waters APC System (Nihon Waters K.K.) |
| ·Detector: | RI, PDA |
| ·Column: | ACQUITY APC XT900 (150 mm), XT200 (75 mm), XT125 (75 mm), XT45 (150 mm) |
| ·Temperature: | 40.0°C |
| ·Solvent: | methanol for high-performance liquid chromatography |
| ·Flow rate: | 0.8 mL/min |
| ·Injection volume: | 10 µL |

### [Method of measuring Amount of Sulfur Element (S) near Surfaces of Resin Particles]

A method of determining the atomic weight ratio of the resin particles in the present disclosure based on ESCA measurement is described. The atomic weight ratio of the resin particles in the present disclosure based on the ESCA measurement is measured by using a product obtained by fixing the resin particles in a freeze-dried state to indium foil. The measurement is performed with the following measuring apparatus under the following measurement conditions.

| | |
|---|---|
| ·Measuring apparatus: | X-ray photoelectron spectrometer Quantum 2000 (product name, manufactured by Ulvac-Phi, Incorporated) |
| ·X-ray source: | monochromatic Al Kα |
| ·X-ray setting: | 100 µmφ (25 W (15 KV)) |
| ·Photoelectron take-off angle: | 45° |
| ·Neutralization condition: | combined use of a neutralization gun and an ion gun |
| ·Analysis region: | 300 µm×200 µm |
| ·Pass energy: | 58.70 eV |
| ·Step size: | 0.125 eV |
| ·Analysis software: | MultiPak (Ulvac-Phi, Incorporated) |

The number of scans at the time of the measurement is as follows: an S2p spectrum is measured 30 times. The resultant quantitative value of the S2p was adopted as the amount of a sulfur element near the surfaces of the resin particles. From the viewpoint of detection accuracy, a detection lower limit was set to 0.05 atm%, and when the amount was less than 0.05 atm%, it was judged that the element was not detected.

### [Method of measuring Number-average Particle Diameter of Resin Particles in Aqueous Dispersion]

A method of measuring the number-average particle diameter of the resin particles each having a sulfur element in the present disclosure in an aqueous dispersion is described. The number-average particle diameter in the aqueous dispersion in the present disclosure is measured under a state in which the resin particles are dispersed in ion-exchanged water so that their concentration may be 0.001 mass%. Ion-exchanged water having an electric conductivity of 10 µS/cm or less is used as the ion-exchanged water. A dynamic light scattering method is applied to the measurement of the number-average particle diameter of the resin particles in the aqueous dispersion. Specifically, the measurement is performed with ZETASIZER (Nano-ZS: Spectris Co., Ltd.) at 25°C. With regard to analysis parameters, the refractive index of a latex (n≈1.59) is selected as the refractive index of each of the particles, and pure water is selected as a dispersion medium. The measurement is performed 10 times, and the average of the 10 measured values is adopted as the number-average particle diameter of the resin particles in the aqueous dispersion.

### [Method of measuring Antibody (Antigen) Sensitization Ratio of Activated Resin Particles]

A method of measuring the antibody (antigen) sensitization ratio of activated resin particles, which are obtained by activating the resin particles in the present disclosure through the sensitization of an antibody or an antigen thereto, is described. The antibody sensitization ratio (%) of the activated resin particles was determined by protein determination. Herein, the term "antibody sensitization ratio (%)" means the ratio of the amount of the antibody bonded to the resin particles to the amount of the antibody used in the reaction (antibody loading amount).

First, 7 mL of the liquid A of PROTEIN ASSAY BCA KIT (Wako Pure Chemical Industries, Ltd.) and 140 µL of the liquid B thereof are mixed, and the prepared liquid is adopted as a liquid AB. Next, 200 µL of the liquid AB is added to 25 µL of the dispersion (0.1% solution) of the activated resin particles (amount of resin particles of 25 µg), and the mixture is incubated at 60°C for 30 minutes. The solution is centrifuged at 4°C and 15,000 rpm (20,400 g) for 5 minutes, and 200 µL of the supernatant is loaded into a 96-well microwell with a pipetter. The absorbance of the supernatant at 562 nm is measured with a microplate reader together with standard samples (several samples obtained by diluting the antibody with 10 mM HEPES so that their concentrations may fall within the range of from 0 µg/mL to 200 µg/mL). The amount of the antibody is calculated from a standard curve. The amount of the antibody sensitized to the resin particles (the amount of the bonded antibody per mg of the resin particles (µg/mg)) is determined by dividing the calculated antibody amount by the mass of the resin particles (in this case, 0.025 mg). Finally, the sensitization ratio is calculated. In the case where the antibody loading amount is 25 µg per mg of the resin particles, when the antibody sensitization amount is 12.5 µg/mg, the sensitization ratio is 50%.

### [Examples]

The present disclosure is described in detail by way of Examples. However, the present disclosure is not limited to these Examples.

Synthesis examples of resin mother particles are described below. The amounts of sulfur elements (S amounts) near the surfaces of the respective resin mother particles measured with an ESCA are shown in Table 1.

### [Resin Mother Particle Synthesis Example]

### (Resin Mother Particles 1)

1.2 Grams of styrene (Kishida Chemical Co., Ltd.), 1.8 g of glycidyl methacrylate (Kishida Chemical Co., Ltd.), 0.04 g of divinylbenzene (Kishida Chemical Co., Ltd.), and 100 g of ion-exchanged water were weighed in a 200-milliliter flask to provide a mixed liquid. After that, while the mixed liquid was stirred at 200 rpm, the mixed liquid was held at 70°C, followed by nitrogen bubbling for 30 minutes. Next, the nitrogen bubbling was switched to a nitrogen flow.

Then, a dissolved liquid separately prepared by dissolving 0.06 g of V-50 (FUJIFILM Wako Pure Chemical Corporation) in 3 g of pure water was added to the above-mentioned mixed liquid to initiate radical polymerization. Two hours after the initiation of the polymerization, 0.3 g of glycidyl methacrylate was added to a radical polymerization reaction field, and the mixture was further held at 70°C for 8 hours while being stirred at 200 rpm. After that, the mixture was slowly cooled to room temperature. At this time point, the contents of the 200-milliliter flask were sampled, and were evaluated for their radical polymerization conversion ratio by using proton NMR, gas chromatography, or gel permeation chromatography. As a result, it was recognized that the ratio was substantially 100%. The polymerization conversion ratio was calculated from the amount of the monomers loaded in the polymerization process. Specifically, the polymerization conversion ratio is a value obtained as follows: the amount of the remaining monomers is determined by analyzing the solution after the polymerization reaction through use of the proton NMR or the like; and a value (polymerization conversion ratio) representing the extent to which the monomers are converted into a polymer by the polymerization reaction is determined from the amount of the remaining monomers.

An aqueous solution prepared in advance by dissolving 3-amino-1,2-propanediol (FUJIFILM Wako Pure Chemical Corporation) and mercaptosuccinic acid (FUJIFILM Wako Pure Chemical Corporation) (whose pH had been adjusted to 7 with triethylamine (Kishida Chemical Co., Ltd.) and a 2 N aqueous solution of hydrochloric acid) was added to the flask. Then, the mixture was held at room temperature for 3 hours while being stirred at 200 rpm. In the above-mentioned aqueous solution, a ratio between 3-amino-1,2-propanediol and mercaptosuccinic acid is 8.5:1.5 (molar ratio). In addition, the total number of the moles of 3-amino-1,2-propanediol and mercaptosuccinic acid is equal to the number of the moles of glycidyl methacrylate. After that, triethylamine was used to adjust the pH of the contents of the 200-milliliter flask to 10. After that, the temperature of the contents was increased to 70°C, and the contents were further held at the temperature for 3 hours while being stirred at 200 rpm. Thus, an epoxy group derived from glycidyl methacrylate, an amino group derived from 3-amino-1,2-propanediol, and a thiol group derived from mercaptosuccinic acid were caused to chemically react with each other to provide resin mother particles 1. During the chemical reaction, no aggregate or the like was formed. The resin mother particles 1 were purified by a centrifugal operation, and their dispersion medium was replaced with a phosphate buffer solution, followed by their storage (the replacement of the dispersion medium was also performed by the centrifugal operation). The resin mother particles 1 were evaluated by using a dynamic light scattering method (DLS-8000: Otsuka Electronics Co., Ltd.). As a result, the particles had a number-average particle diameter of 238 nm.

### (Resin Mother Particles 2)

Resin mother particles 2 were produced by the same operation as that of the resin mother particles 1 except that the molar ratio between 3-amino-1,2-propanediol and mercaptosuccinic acid was changed to 7:3. The resin mother particles 2 were evaluated by using a dynamic light scattering method (DLS-8000: Otsuka Electronics Co., Ltd.). As a result, the particles had a number-average particle diameter of 235 nm.

### (Resin Mother Particles 3)

Resin mother particles 3 were produced by the same operation as that of the resin mother particles 1 except that: 3-mercapto-1,2-propanediol was used instead of 3-amino-1,2-propanediol; and a molar ratio between 3-mercapto-1,2-propanediol and mercaptosuccinic acid was set to 9:1. The resin mother particles 3 were evaluated by using a dynamic light scattering method (DLS-8000: Otsuka Electronics Co., Ltd.). As a result, the particles had a number-average particle diameter of 236 nm.

### (Resin Mother Particles 4)

Resin mother particles 4 were produced by the same operation as that of the resin mother particles 1 except that: 3-mercapto-1,2-propanediol was used instead of 3-amino-1,2-propanediol; aminosuccinic acid was used instead of mercaptosuccinic acid; and a molar ratio between 3-mercapto-1,2-propanediol and aminosuccinic acid was set to 1.5:8.5. The resin mother particles 4 were evaluated by using a dynamic light scattering method (DLS-8000: Otsuka Electronics Co., Ltd.). As a result, the particles had a number-average particle diameter of 233 nm.

### (Resin Mother Particles 5)

1.2 Grams of styrene (Kishida Chemical Co., Ltd.), 0.02 g of acrylic acid (FUJIFILM Wako Pure Chemical Corporation), 0.04 g of divinylbenzene (Kishida Chemical Co., Ltd.), and 100 g of ion-exchanged water were weighed in a 200-milliliter flask to provide a mixed liquid. After that, while the mixed liquid was stirred at 200 rpm, the mixed liquid was held at 70°C, followed by nitrogen bubbling for 30 minutes. Next, the nitrogen bubbling was switched to a nitrogen flow.

Then, a dissolved liquid separately prepared by dissolving 0.06 g of potassium peroxodisulfate (FUJIFILM Wako Pure Chemical Corporation) in 3 g of pure water was added to the above-mentioned mixed liquid to initiate radical polymerization. The mixture was held at 85°C for 6 hours after the initiation of the polymerization while being stirred at 200 rpm. After that, the mixture was slowly cooled to room temperature. At this time point, the contents of the 200-milliliter flask were sampled, and were evaluated for their radical polymerization conversion ratio by using proton NMR, gas chromatography, or gel permeation chromatography. As a result, it was recognized that the ratio was substantially 100%, and resin mother particles 5 were obtained. During the chemical reaction, no aggregate or the like was formed. The resin mother particles 5 were purified by a centrifugal operation, and their dispersion medium was replaced with a phosphate buffer solution, followed by their storage (the replacement of the dispersion medium was also performed by the centrifugal operation). The resin mother particles 5 were evaluated by using a dynamic light scattering method (DLS-8000: Otsuka Electronics Co., Ltd.). As a result, the particles had a number-average particle diameter of 218 nm.

### (Resin Mother Particles 6)

Resin mother particles 6 were produced by the same operation as that of the resin mother particles 5 except that the amount of potassium peroxodisulfate was changed to 0.03 g. The resin mother particles 6 were evaluated by using a dynamic light scattering method (DLS-8000: Otsuka Electronics Co., Ltd.). As a result, the particles had a number-average particle diameter of 227 nm.

### (Resin Mother Particles 7)

1.2 Grams of styrene (Kishida Chemical Co., Ltd.), 1.8 g of glycidyl methacrylate (Kishida Chemical Co., Ltd.), 0.04 g of divinylbenzene (Kishida Chemical Co., Ltd.), 0.05 g of sodium styrenesulfonate, and 100 g of ion-exchanged water were weighed in a 200-milliliter flask to provide a mixed liquid. After that, while the mixed liquid was stirred at 200 rpm, the mixed liquid was held at 70°C, followed by nitrogen bubbling for 30 minutes. Next, the nitrogen bubbling was switched to a nitrogen flow.

Then, a dissolved liquid separately prepared by dissolving 0.02 g of potassium peroxodisulfate (FUJIFILM Wako Pure Chemical Corporation) in 3 g of pure water was added to the above-mentioned mixed liquid to initiate radical polymerization. Two hours after the initiation of the polymerization, 0.3 g of glycidyl methacrylate was added to a radical polymerization reaction field, and the mixture was further held at 70°C for 8 hours while being stirred at 200 rpm. After that, the mixture was slowly cooled to room temperature. At this time point, the contents of the 200-milliliter flask were sampled, and were evaluated for their radical polymerization conversion ratio by using proton NMR, gas chromatography, or gel permeation chromatography. As a result, it was recognized that the ratio was substantially 100%.

An aqueous solution of 3-amino-1,2-propanediol prepared in advance (whose pH had been adjusted to 7 with triethylamine (Kishida Chemical Co., Ltd.) and a 2 N aqueous solution of hydrochloric acid) was added to the flask. Then, the mixture was held at room temperature for 3 hours while being stirred at 200 rpm. In the above-mentioned aqueous solution, the number of the moles of 3-amino-1,2-propanediol is equal to the number of the moles of glycidyl methacrylate. After that, triethylamine was used to adjust the pH of the contents of the 200-milliliter flask to 10. After that, the temperature of the contents was increased to 70°C, and the contents were further held at the temperature for 3 hours while being stirred at 200 rpm. Thus, an epoxy group derived from glycidyl methacrylate and an amino group derived from 3-amino-1,2-propanediol were caused to chemically react with each other to provide resin mother particles 7. During the chemical reaction, no aggregate or the like was formed. The resin mother particles 7 were purified by a centrifugal operation, and their dispersion medium was replaced with a phosphate buffer solution containing 3% BSA, followed by their storage (the replacement of the dispersion medium was also performed by the centrifugal operation). The resin mother particles 7 were evaluated by using a dynamic light scattering method (DLS-8000: Otsuka Electronics Co., Ltd.). As a result, the particles had a number-average particle diameter of 238 nm.

### (Resin Mother Particles 8)

Resin mother particles 8 were produced by the same operation as that of the resin mother particles 7 except that the amount of sodium styrenesulfonate was changed from 0.05 g to 0.075 g. The resin mother particles 8 were evaluated by using a dynamic light scattering method (DLS-8000: Otsuka Electronics Co., Ltd.). As a result, the particles had a number-average particle diameter of 234 nm.

### (Resin Mother Particles 9)

Resin mother particles 9 were produced by the same operation as that of the resin mother particles 7 except that the amount of sodium styrenesulfonate was changed from 0.05 g to 0.14 g. The resin mother particles 9 were evaluated by using a dynamic light scattering method (DLS-8000: Otsuka Electronics Co., Ltd.). As a result, the particles had a number-average particle diameter of 228 nm.

### (Resin Mother Particles 10)

Resin mother particles 10 were produced by the same operation as that of the resin mother particles 7 except that the amount of sodium styrenesulfonate was changed from 0.05 g to 0.16 g. The resin mother particles 10 were evaluated by using a dynamic light scattering method (DLS-8000: Otsuka Electronics Co., Ltd.). As a result, the particles had a number-average particle diameter of 228 nm.

### (Resin Mother Particles 11)

Resin mother particles 11 were produced by the same operation as that of the resin mother particles 7 except that the amount of sodium styrenesulfonate was changed from 0.05 g to 0.20 g. The resin mother particles 11 were evaluated by using a dynamic light scattering method (DLS-8000: Otsuka Electronics Co., Ltd.). As a result, the particles had a number-average particle diameter of 216 nm.

### (Resin Mother Particles 12)

Resin mother particles 12 were produced by the same operation as that of the resin mother particles 7 except that the amount of sodium styrenesulfonate was changed from 0.05 g to 0.0035 g. The resin mother particles 12 were evaluated by using a dynamic light scattering method (DLS-8000: Otsuka Electronics Co., Ltd.). As a result, the particles had a number-average particle diameter of 228 nm.

### (Resin Mother Particles 13)

3.0 Grams of styrene (Kishida Chemical Co., Ltd.), 0.04 g of divinylbenzene (Kishida Chemical Co., Ltd.), 0.02 g of sodium styrenesulfonate, and 100 g of ion-exchanged water were weighed in a 200-milliliter flask to provide a mixed liquid. After that, while the mixed liquid was stirred at 200 rpm, the mixed liquid was held at 70°C, followed by nitrogen bubbling for 30 minutes. Next, the nitrogen bubbling was switched to a nitrogen flow.

Then, a dissolved liquid separately prepared by dissolving 0.03 g of potassium peroxodisulfate (FUJIFILM Wako Pure Chemical Corporation) in 3 g of pure water was added to the above-mentioned mixed liquid to initiate radical polymerization. The mixture was held at 70°C for 10 hours while being stirred at 200 rpm. After that, the mixture was slowly cooled to room temperature. At this time point, the contents of the 200-milliliter flask were sampled, and were evaluated for their radical polymerization conversion ratio by using proton NMR, gas chromatography, or gel permeation chromatography. As a result, it was recognized that the ratio was substantially 100%. During the chemical reaction, no aggregate or the like was formed. The resin mother particles 13 were purified by a centrifugal operation, and their dispersion medium was replaced with a phosphate buffer solution containing 3% BSA, followed by their storage (the replacement of the dispersion medium was also performed by the centrifugal operation). The resin mother particles 13 were evaluated by using a dynamic light scattering method (DLS-8000: Otsuka Electronics Co., Ltd.). As a result, the particles had a number-average particle diameter of 224 nm.

### (Resin Mother Particles 14)

1.2 Grams of styrene (Kishida Chemical Co., Ltd.), 1.8 g of glycidyl methacrylate (Kishida Chemical Co., Ltd.), 0.04 g of divinylbenzene (Kishida Chemical Co., Ltd.), and 100 g of ion-exchanged water were weighed in a 200-milliliter flask to provide a mixed liquid. After that, while the mixed liquid was stirred at 200 rpm, the mixed liquid was held at 70°C, followed by nitrogen bubbling for 30 minutes. Next, the nitrogen bubbling was switched to a nitrogen flow.

Then, a dissolved liquid separately prepared by dissolving 0.06 g of V-50 (FUJIFILM Wako Pure Chemical Corporation) in 3 g of pure water was added to the above-mentioned mixed liquid to initiate radical polymerization. Two hours after the initiation of the polymerization, 0.3 g of glycidyl methacrylate was added to a radical polymerization reaction field, and the mixture was further held at 70°C for 8 hours while being stirred at 200 rpm. After that, the mixture was slowly cooled to room temperature. At this time point, the contents of the 200-milliliter flask were sampled, and were evaluated for their radical polymerization conversion ratio by using proton NMR, gas chromatography, or gel permeation chromatography. As a result, it was recognized that the ratio was substantially 100%.

Next, an aqueous solution prepared in advance by dissolving glycine (aqueous solution in which the ratio of the number of the moles of glycine to the number of the moles of glycidyl methacrylate was 1/10 mol, and whose pH had been adjusted to 7 with triethylamine (Kishida Chemical Co., Ltd.) and a 2 N aqueous solution of hydrochloric acid) was added to the flask. After that, the mixture was held at room temperature for 3 hours while being stirred at 200 rpm. Triethylamine was used to adjust the pH of the contents of the 200-milliliter flask to 10. After that, the temperature of the contents was increased to 70°C, and the contents were further held at the temperature for 3 hours while being stirred at 200 rpm. Thus, an epoxy group derived from glycidyl methacrylate and an amino group derived from glycine were caused to chemically react with each other. Next, a 2 N aqueous solution of hydrochloric acid was used to adjust the pH of the contents of the 200-milliliter flask to 1.5, and the contents were further held at 70°C for 3 hours while being stirred at 200 rpm. Thus, an unreacted epoxy group was caused to chemically react with water so that the epoxy group was converted into a glycol. As a result, resin mother particles 14 each having a carboxy group as a reactive functional group were obtained. During the chemical reaction, an aggregate was formed, and hence the aggregate was removed by decantation. After that, the resin mother particles 14 were purified by a centrifugal operation, and their dispersion medium was replaced with a phosphate buffer solution, followed by their storage (the replacement of the dispersion medium was also performed by the centrifugal operation). The resin mother particles 14 were evaluated by using a dynamic light scattering method (DLS-8000: Otsuka Electronics Co., Ltd.). As a result, the particles had a number-average particle diameter of 222 nm.

Production examples of dispersions of activated resin particles are described below. The sensitization ratio of an antibody used in the activation is shown in Table 1.

### [Production Example of Dispersion of Activated Resin Particles activated by Chemical Adsorption]

180 Microliters of a 0.017 mass% water suspension of each of the resin mother particles 1 to 6 and 14 was dispensed in a 1.5-milliliter microtube. 90 Microliters of a 0.1% aqueous solution of 1-(3-dimethylaminopropyl)-3-ethylcarbodiimide hydrochloride and 90 µL of a 0.1% aqueous solution of N-hydroxysulfosuccinimide sodium salt were added thereto. The mixture was stirred at room temperature for 30 minutes to provide a dispersion of resin particles each having an activated carboxy group.

After centrifugal washing, 270 µL of 10 mM HEPES having a pH of 7.2 was added to the resultant, and the resin particles each having an activated carboxy group were dispersed with an ultrasonic wave.

5 Microliters of a 4.9 mg/mL dispersion of clone C5 (Funakoshi Co., Ltd.) of a monoclonal mouse anti-human C-reactive protein (hereinafter referred to as "CRP antibody") was added thereto. The mixture was stirred at room temperature for 3 hours to provide resin particles activated by antibody sensitization to the resin particles each having an activated carboxy group (hereinafter referred to as "activated resin particles"). Those activated resin particles of each kind were subjected to centrifugal washing, and were then dispersed in a 10 mM HEPES buffer solution having a pH of 7.9, the solution having added thereto 0.05% Tween 20, to produce an activated resin particle dispersion. The sensitization ratios of the CRP antibody to the respective activated resin particles are shown in Table 1. An activated resin particle dispersion 14 was prepared by further adding 0.5 mg/mL BSA because the dispersibility of its corresponding activated resin particles was insufficient.

### [Production Example of Dispersion of Activated Resin Particles activated by Physical Adsorption]

A liquid obtained by dispersing each of the resin mother particles 7 to 13 in 270 µL of 10 mM HEPES having a pH of 7.2 so that a particle concentration became 0.017 mass% was dispensed in a 1.5-milliliter microtube, and 5 µL of a 4.9 mg/mL dispersion of the clone C5 (Funakoshi Co., Ltd.) of the CRP antibody was added thereto, followed by stirring at room temperature for 1 hour. Those resin mother particles were subjected to centrifugal washing, and were then subjected to blocking treatment by being mixed with a 1 mg/mL aqueous solution of BSA. Thus, activated resin particles were obtained. The activated resin particles were subjected to centrifugal washing, and then activated resin particle dispersions 7 to 13 were each produced. The sensitization ratios of the CRP antibody to the respective activated resin particles are shown in Table 1. To evaluate the sensitization ratio of the CRP antibody, a resin particle dispersion was simultaneously produced by performing the same operation without mixing the CRP antibody.

**Table 1**

| Activated resin particle dispersion | Resin mother particles | Surface S amount determined with ESCA (%) | Activation approach | Antibody sensitization ratio |
|---|---|---|---|---|
| Activated resin particle dispersion 1 | Resin mother particles 1 | 1.50 | Chemical adsorption | 93% |
| Activated resin particle dispersion 2 | Resin mother particles 2 | 2.00 | Chemical adsorption | 92% |
| Activated resin particle dispersion 3 | Resin mother particles 3 | 3.50 | Chemical adsorption | 94% |
| Activated resin particle dispersion 4 | Resin mother particles 4 | 0.55 | Chemical adsorption | 88% |
| Activated resin particle dispersion 5 | Resin mother particles 5 | 0.25 | Chemical adsorption | 87% |
| Activated resin particle dispersion 6 | Resin mother particles 6 | 0.07 | Chemical adsorption | 91% |
| Activated resin particle dispersion 7 | Resin mother particles 7 | 2.00 | Physical adsorption | 89% |
| Activated resin particle dispersion 8 | Resin mother particles 8 | 3.20 | Physical adsorption | 93% |
| Activated resin particle dispersion 9 | Resin mother particles 9 | 5.80 | Physical adsorption | 89% |
| Activated resin particle dispersion 10 | Resin mother particles 10 | 7.20 | Physical adsorption | 90% |
| Activated resin particle dispersion 11 | Resin mother particles 11 | 8.70 | Physical adsorption | 87% |
| Activated resin particle dispersion 12 | Resin mother particles 12 | 0.40 | Physical adsorption | 83% |
| Activated resin particle dispersion 13 | Resin mother particles 13 | 2.90 | Physical adsorption | 86% |
| Activated resin particle dispersion 14 | Resin mother particles 14 | 0.00 | Chemical adsorption | 87% |

### [Production of Buffer Solutions 1 to 26]

Products obtained by mixing predetermined amounts of the respective buffers and additives as shown in Table 2 (Table 2-1 and Table 2-2) were adopted as buffer solutions 1 to 26. The pHs of the respective buffer solutions are also shown in Table 2-1 and Table 2-2. An aqueous solution of sodium hydroxide or an aqueous solution of hydrochloric acid was used in pH adjustment.

**Table 2-1**

| Buffer solution | Buffer | | Additive 1 | |
|---|---|---|---|---|
| | Kind | Concentration (M) | Kind | Concentration (M) |
| Buffer solution 1 | HEPES | 0.05 | Thiourea | 0.5 |
| Buffer solution 2 | MOPS | 0.05 | Thiourea | 0.5 |
| Buffer solution 3 | MES | 0.05 | Thiourea | 0.5 |
| Buffer solution 4 | Bis-Tris | 0.05 | Thiourea | 0.5 |
| Buffer solution 5 | HEPES | 0.05 | Thiourea | 0.5 |
| Buffer solution 6 | HEPES | 0.05 | 1,3-Diethyl-2-thiourea | 0.1 |
| Buffer solution 7 | HEPES | 0.05 | 1,3-Dibutyl-2-thiourea | 0.05 |
| Buffer solution 8 | PBS | 0.05 | Thiourea | 0.5 |
| Buffer solution 9 | HEPES | 0.05 | Thiourea | 0.5 |
| Buffer solution 10 | HEPES | 0.05 | Thiourea | 0.01 |
| Buffer solution 11 | HEPES | 0.05 | Thiourea | 0.001 |
| Buffer solution 12 | HEPES | 0.05 | Thiourea | 1 |
| Buffer solution 13 | HEPES | 0.05 | Thiourea | 3 |
| Buffer solution 14 | MES | 0.05 | Thiourea | 0.5 |
| Buffer solution 15 | MES | 0.05 | Thiourea | 0.5 |
| Buffer solution 16 | PBS | 0.05 | Thiourea | 0.5 |
| Buffer solution 17 | PBS | 0.05 | Thiourea | 0.5 |
| Buffer solution 18 | CHES | 0.05 | Thiourea | 0.5 |
| Buffer solution 19 | CHES | 0.05 | Thiourea | 0.5 |
| Buffer solution 20 | Tris | 0.05 | Thiourea | 0.5 |
| Buffer solution 21 | Tris | 0.05 | Thiourea | 0.5 |
| Buffer solution 22 | PBS | 0.05 | 1,3-Diethyl-2-thiourea | 0.1 |
| Buffer solution 23 | HEPES | 0.05 | - | - |
| Buffer solution 24 | PBS | 0.05 | Urea | 0.5 |
| Buffer solution 25 | PBS | 0.05 | 2-Mercaptoethanol | 0.00001 |
| Buffer solution 26 | PBS | 0.001 | - | - |

**Table 2-2**

| Buffer solution | Additive 2 | | Additive 3 | | pH |
|---|---|---|---|---|---|
| | Kind | Concentration (M) | Kind | Addition amount (mass%) | |
| Buffer solution 1 | EDTA-4Na | 0.01 | Tween 20 | 0.01 | 7.2 |
| Buffer solution 2 | EDTA-4Na | 0.01 | Tween 20 | 0.01 | 7.0 |
| Buffer solution 3 | EDTA-4Na | 0.01 | Tween 20 | 0.01 | 6.8 |
| Buffer solution 4 | EDTA-4Na | 0.01 | Tween 20 | 0.01 | 7.1 |
| Buffer solution 5 | EDTA-2Na | 0.01 | Tween 20 | 0.01 | 7.2 |
| Buffer solution 6 | EDTA-4Na | 0.01 | Tween 20 | 0.01 | 7.2 |
| Buffer solution 7 | EDTA-4Na | 0.01 | Tween 20 | 0.01 | 7.2 |
| Buffer solution 8 | EDTA-4Na | 0.01 | Tween 20 | 0.01 | 7.2 |
| Buffer solution 9 | - | - | Tween 20 | 0.01 | 7.2 |
| Buffer solution 10 | EDTA-4Na | 0.01 | Tween 20 | 0.01 | 7.2 |
| Buffer solution 11 | EDTA-4Na | 0.01 | Tween 20 | 0.01 | 7.2 |
| Buffer solution 12 | EDTA-4Na | 0.01 | Tween 20 | 0.01 | 7.2 |
| Buffer solution 13 | EDTA-4Na | 0.01 | Tween 20 | 0.01 | 7.2 |
| Buffer solution 14 | EDTA-4Na | 0.01 | Tween 20 | 0.01 | 6.5 |
| Buffer solution 15 | EDTA-4Na | 0.01 | Tween 20 | 0.01 | 6.0 |
| Buffer solution 16 | EDTA-4Na | 0.01 | Tween 20 | 0.01 | 6.0 |
| Buffer solution 17 | EDTA-4Na | 0.01 | Tween 20 | 0.01 | 5.7 |
| Buffer solution 18 | EDTA-4Na | 0.01 | Tween 20 | 0.01 | 8.5 |
| Buffer solution 19 | EDTA-4Na | 0.01 | Tween 20 | 0.01 | 9.0 |
| Buffer solution 20 | EDTA-4Na | 0.01 | Tween 20 | 0.01 | 9.0 |
| Buffer solution 21 | EDTA-4Na | 0.01 | Tween 20 | 0.01 | 9.3 |
| Buffer solution 22 | - | - | Tween 20 | 0.01 | 7.2 |
| Buffer solution 23 | EDTA-4Na | 0.01 | Tween 20 | 0.01 | 7.2 |
| Buffer solution 24 | EDTA-4Na | 0.01 | Tween 20 | 0.01 | 7.2 |
| Buffer solution 25 | EDTA-4Na | 0.01 | Tween 20 | 0.01 | 7.2 |
| Buffer solution 26 | - | - | Tween 20 | 0.01 | 7.2 |

### [Performance Evaluation]

Evaluation 1 and Evaluation 2 to be described later were performed by using the resin mother particles, the activated resin particle dispersions, and the buffer solutions described in the above-mentioned production examples.

The mass ratio W(A)/W(B) of the content W(A) of a thiourea compound to the content W(B) of resin particles in the whole amount of each of the particle dispersions and the buffer solutions is shown in each of Table 3 and Table 4.

### (Evaluation 1: Evaluation of Nonspecific Adsorptivity to Human Normal Specimen)

A dispersion (liquid P) was prepared by dispersing each of the resin mother particles 1 to 14 in 10 mM HEPES having a pH of 7.9 (containing 0.05% Tween 20) so that the concentration of the particles became 0.1 mass%. Next, 55 µL of each of a diluted specimen liquid (liquid Q), which was formed of a human normal specimen A (serum specimen, 5 µL) and any one of the respective buffer solutions 1 to 25 shown in Table 2-1 and Table 2-2 (50 µL), and a diluted specimen liquid (liquid Q'), which was formed of the human normal specimen A (serum specimen, 5 µL) and the buffer solution 26 (50 µL), was prepared.

Next, with regard to each of Examples 1 to 32 and Comparative Examples 1 to 4, a mixed liquid obtained by stirring the liquid Q or the liquid Q' was left to stand still at 37°C for 5 minutes. After the still standing, the liquid P (50 µL) was added to the liquid Q (55 µL) or the liquid Q' (55 µL), and immediately after that, the absorbance of the mixture at a wavelength of 572 nm was measured. Herein, a mixed liquid obtained by adding the liquid P to the liquid Q was adopted as a liquid PQ, and a mixed liquid obtained by adding the liquid P to the liquid Q' was adopted as a liquid PQ'. After that, each of the mixed liquids was further left to stand still at 37°C for 5 minutes, and then its absorbance at a wavelength of 572 nm was measured again, followed by the calculation of its absorbance variation ΔABS.

With regard to each of Examples 33 and 34, the liquid P (50 µL) and the liquid Q (55 µL) or the liquid Q' (55 µL) were mixed in advance at 25°C, and immediately after that, the absorbance of the mixture was measured. After that, the mixture was further left to stand still at 37°C for 5 minutes, and then its absorbance at a wavelength of 572 nm was measured again, followed by the calculation of its absorbance variation ΔABS.

A spectrophotometer BioSpectrometer manufactured by Eppendorf SE was used in the absorbance measurement. The extent to which the ΔOD of the liquid PQ reduced as compared to the ΔOD of the liquid PQ' free of an additive except Tween 20 was evaluated by a reduction ratio. The ratio was used to perform a comparative evaluation on whether or not nonspecific agglutination between the specimen and the resin mother particles was able to be suppressed by the combination of each resin mother particle dispersion and a buffer solution containing each additive. The liquid PQ having a large value of the reduction ratio is interpreted as being suppressed in nonspecific adsorption. When the liquid PQ in which the nonspecific agglutination is large is used as a test reagent for a latex agglutination method in a specimen test, concern is raised in that the normal specimen is interpreted as being false positive. A threshold shown in Table 3 is a criterion determined from a noise risk at the time of an attempt to detect a low-concentration target substance by the latex agglutination method, and hence in the case of a rank E, that is, an improvement ratio of less than 10%, concern is raised in that the normal specimen is interpreted as being false positive. It was recognized by a pre-evaluation that the human normal specimen A used in Evaluation 1 this time nonspecifically adsorbed to each of resin mother particles each having a sulfur element.

In addition, the same rank tendency as that of Evaluation 1 was observed even in each of the activated resin particle dispersions each having sensitized thereto the antibody.

| | |
|---|---|
| A: | 70% or more |
| B: | 50% or more and less than 70% |
| C: | 30% or more and less than 50% |
| D: | 10% or more and less than 30% |
| E: | Less than 10% |

**Table 3**

| Example | Resin mother particles for liquid P | Buffer solution for liquid Q | Buffer solution for liquid Q' | W(A)/W(B) | Evaluation 1 Evaluation of nonspecific adsorptivity to human normal specimen A |
|---|---|---|---|---|---|
| | | | | | Rank |
| Example 1 | Resin mother particles 1 | Buffer solution 1 | Buffer solution 26 | 76 | A |
| Example 2 | Resin mother particles 2 | Buffer solution 1 | Buffer solution 26 | 76 | A |
| Example 3 | Resin mother particles 3 | Buffer solution 1 | Buffer solution 26 | 76 | A |
| Example 4 | Resin mother particles 3 | Buffer solution 2 | Buffer solution 26 | 76 | A |
| Example 5 | Resin mother particles 3 | Buffer solution 3 | Buffer solution 26 | 76 | A |
| Example 6 | Resin mother particles 3 | Buffer solution 4 | Buffer solution 26 | 76 | A |
| Example 7 | Resin mother particles 3 | Buffer solution 5 | Buffer solution 26 | 76 | A |
| Example 8 | Resin mother particles 3 | Buffer solution 6 | Buffer solution 26 | 15.2 | B |
| Example 9 | Resin mother particles 3 | Buffer solution 7 | Buffer solution 26 | 7.6 | C |
| Example 10 | Resin mother particles 3 | Buffer solution 8 | Buffer solution 26 | 76 | B |
| Example 11 | Resin mother particles 3 | Buffer solution 9 | Buffer solution 26 | 76 | B |
| Example 12 | Resin mother particles 3 | Buffer solution 10 | Buffer solution 26 | 1.52 | B |
| Example 13 | Resin mother particles 3 | Buffer solution 11 | Buffer solution 26 | 0.152 | C |
| Example 14 | Resin mother particles 3 | Buffer solution 12 | Buffer solution 26 | 152 | A |
| Example 15 | Resin mother particles 3 | Buffer solution 13 | Buffer solution 26 | 456 | A |
| Example 16 | Resin mother particles 3 | Buffer solution 14 | Buffer solution 26 | 76 | B |
| Example 17 | Resin mother particles 3 | Buffer solution 15 | Buffer solution 26 | 76 | B |
| Example 18 | Resin mother particles 3 | Buffer solution 16 | Buffer solution 26 | 76 | C |
| Example 19 | Resin mother particles 3 | Buffer solution 17 | Buffer solution 26 | 76 | C |
| Example 20 | Resin mother particles 3 | Buffer solution 18 | Buffer solution 26 | 76 | B |
| Example 21 | Resin mother particles 3 | Buffer solution 19 | Buffer solution 26 | 76 | B |
| Example 22 | Resin mother particles 3 | Buffer solution 20 | Buffer solution 26 | 76 | C |
| Example 23 | Resin mother particles 3 | Buffer solution 21 | Buffer solution 26 | 76 | C |
| Example 24 | Resin mother particles 4 | Buffer solution 1 | Buffer solution 26 | 76 | A |
| Example 25 | Resin mother particles 5 | Buffer solution 1 | Buffer solution 26 | 76 | B |
| Example 26 | Resin mother particles 6 | Buffer solution 1 | Buffer solution 26 | 76 | C |
| Example 27 | Resin mother particles 7 | Buffer solution 1 | Buffer solution 26 | 76 | C |
| Example 28 | Resin mother particles 8 | Buffer solution 1 | Buffer solution 26 | 76 | B |
| Example 29 | Resin mother particles 9 | Buffer solution 1 | Buffer solution 26 | 76 | B |
| Example 30 | Resin mother particles 10 | Buffer solution 1 | Buffer solution 26 | 76 | C |
| Example 31 | Resin mother particles 11 | Buffer solution 1 | Buffer solution 26 | 76 | D |
| Example 32 | Resin mother particles 12 | Buffer solution 1 | Buffer solution 26 | 76 | C |
| Example 33 | Resin mother particles 3 | Buffer solution 1 | Buffer solution 26 | 76 | B |
| Example 34 | Resin mother particles 5 | Buffer solution 22 | Buffer solution 26 | 15.2 | D |
| Comparative Example 1 | Resin mother particles 13 | Buffer solution 23 | Buffer solution 26 | 0 | E |
| Comparative Example 2 | Resin mother particles 13 | Buffer solution 24 | Buffer solution 26 | 0 | E |
| Comparative Example 3 | Resin mother particles 13 | Buffer solution 25 | Buffer solution 26 | 0 | E |
| Comparative Example 4 | Resin mother particles 14 | Buffer solution 6 | Buffer solution 26 | - | E |

### (Evaluation 2: Evaluation of Standard Serum)

A product obtained by diluting standard serum for CRP with a HEPES buffer having a pH of 7.2 so that its concentration became 0.75 mg/dL was adopted as a CRP specimen solution. 1 Microliter of the CRP specimen solution and 50 µL of each buffer solution used in the preparation of the liquid Q of Evaluation 1 were mixed to prepare a mixed liquid (hereinafter referred to as "R1+"), and its temperature was kept at 37°C. In addition, 1 µL of the HEPES buffer having a pH of 7.2 and 50 µL of each buffer solution used in the preparation of the liquid Q of Evaluation 1 were mixed to prepare a mixed liquid (hereinafter referred to as "R1-") as a control, and its temperature was similarly kept at 37°C.

Next, 50 µL of a product (particle concentration: 0.1 mass%, referred to as "R2") obtained by sufficiently dispersing the particles in each of the activated resin particle dispersions 1 to 14 shown in Table 1 with an ultrasonic wave again before its use and the R1+ were mixed. Similarly, the R2 and the R1- were mixed.

The absorbance at a wavelength of 572 nm of the mixed liquid (volume: 101 µL) immediately after stirring was measured. A spectrophotometer BioSpectrometer manufactured by Eppendorf SE was used in the absorbance measurement. Then, the mixed liquid was left to stand still at 37°C for 5 minutes, and then its absorbance at a wavelength of 572 nm was measured again, followed by the calculation of the value of "absorbance variation ΔABS×10,000."

A larger value of the absorbance variation for the R1- means that agglutination resulting from nonspecific adsorption or osmotic pressure agglutination occurs in the resin particles to a larger extent. In this case, when the resin particles are used as particles for a latex agglutination method in a specimen test, concern is raised in that the normal specimen is interpreted as being false positive owing to noise. It was recognized that such agglutination was not observed in each of combinations evaluated in Examples, the combinations including the activated resin particles (activated resin particle dispersions 1 to 12) and the buffer solutions (buffer solutions 1 to 22). Meanwhile, in each of combinations evaluated in Comparative Examples, the combinations each including the activated resin particles (activated resin particle dispersion 14) or any one of the buffer solutions (buffer solutions 23 to 25), the value was large and hence the agglutination was observed.

Resin particles, which show a larger value of the absorbance variation when mixed with the R1+, are expected to be capable of detecting a low-concentration target substance with higher sensitivity when used as particles for a latex agglutination method in a specimen test. The results are summarized in Table 4. In the case of a rank E, that is, an absorbance variation of less than 1,000, concern is raised in that the low-concentration target substance cannot be sufficiently detected, and hence an abnormal specimen is interpreted as being false negative.

| | |
|---|---|
| AA: | 1,750 or more |
| A: | 1,500 or more and less than 1,750 |
| B: | 1,350 or more and less than 1,500 |
| C: | 1,200 or more and less than 1,350 |
| **D:** | 1,000 or more and less than 1,200 |
| **E:** | Less than 1,000 |

**Table 4**

| Example | Resin particle dispersion for R2 | Buffer solution for R1+/R1- | W(A)/W(B) | Evaluation 2 Evaluation of standard serum |
|---|---|---|---|---|
| | | | | Rank |
| Example 35 | Activated resin particle dispersion 1 | Buffer solution 1 | 76 | AA |
| Example 36 | Activated resin particle dispersion 2 | Buffer solution 1 | 76 | AA |
| Example 37 | Activated resin particle dispersion 3 | Buffer solution 1 | 76 | A |
| Example 38 | Activated resin particle dispersion 3 | Buffer solution 2 | 76 | A |
| Example 39 | Activated resin particle dispersion 3 | Buffer solution 3 | 76 | A |
| Example 40 | Activated resin particle dispersion 3 | Buffer solution 4 | 76 | A |
| Example 41 | Activated resin particle dispersion 3 | Buffer solution 5 | 76 | A |
| Example 42 | Activated resin particle dispersion 3 | Buffer solution 6 | 15.2 | B |
| Example 43 | Activated resin particle dispersion 3 | Buffer solution 7 | 7.6 | B |
| Example 44 | Activated resin particle dispersion 3 | Buffer solution 8 | 76 | A |
| Example 45 | Activated resin particle dispersion 3 | Buffer solution 9 | 76 | A |
| Example 46 | Activated resin particle dispersion 3 | Buffer solution 10 | 1.52 | A |
| Example 47 | Activated resin particle dispersion 3 | Buffer solution 11 | 0.152 | A |
| Example 48 | Activated resin particle dispersion 3 | Buffer solution 12 | 152 | B |
| Example 49 | Activated resin particle dispersion 3 | Buffer solution 13 | 456 | C |
| Example 50 | Activated resin particle dispersion 3 | Buffer solution 14 | 76 | A |
| Example 51 | Activated resin particle dispersion 3 | Buffer solution 15 | 76 | B |
| Example 52 | Activated resin particle dispersion 3 | Buffer solution 16 | 76 | B |
| Example 53 | Activated resin particle dispersion 3 | Buffer solution 17 | 76 | C |
| Example 54 | Activated resin particle dispersion 3 | Buffer solution 18 | 76 | A |
| Example 55 | Activated resin particle dispersion 3 | Buffer solution 19 | 76 | B |
| Example 56 | Activated resin particle dispersion 3 | Buffer solution 20 | 76 | B |
| Example 57 | Activated resin particle dispersion 3 | Buffer solution 21 | 76 | C |
| Example 58 | Activated resin particle dispersion 4 | Buffer solution 1 | 76 | A |
| Example 59 | Activated resin particle dispersion 5 | Buffer solution 1 | 76 | C |
| Example 60 | Activated resin particle dispersion 6 | Buffer solution 1 | 76 | D |
| Example 61 | Activated resin particle dispersion 7 | Buffer solution 1 | 76 | B |
| Example 62 | Activated resin particle dispersion 8 | Buffer solution 1 | 76 | B |
| Example 63 | Activated resin particle dispersion 9 | Buffer solution 1 | 76 | B |
| Example 64 | Activated resin particle dispersion 10 | Buffer solution 1 | 76 | C |
| Example 65 | Activated resin particle dispersion 11 | Buffer solution 1 | 76 | C |
| Example 66 | Activated resin particle dispersion 12 | Buffer solution 1 | 76 | C |
| Example 67 | Activated resin particle dispersion 5 | Buffer solution 22 | 15.2 | D |
| Comparative Example 5 | Activated resin particle dispersion 13 | Buffer solution 23 | 0 | E |
| Comparative Example 6 | Activated resin particle dispersion 13 | Buffer solution 24 | 0 | E |
| Comparative Example 7 | Activated resin particle dispersion 13 | Buffer solution 25 | 0 | E |
| Comparative Example 8 | Activated resin particle dispersion 14 | Buffer solution 6 | - | E |

While the present disclosure has been described with reference to embodiments, it is to be understood that the present disclosure is not limited to the disclosed embodiments. The scope of the following claims is to be accorded the broadest interpretation so as to encompass all such modifications and equivalent structures and functions.

Various embodiments have been described in detail above but it will be understood that the present disclosure is not limited to these embodiments and encompasses all modifications, variants, alternatives and equivalents falling within the scope of the appended claims.

## Claims

1. A test reagent for in vitro diagnosis comprising:
a thiourea compound; and
resin particles,
wherein the resin particles each have a sulfur element.

2. The test reagent according to claim 1, wherein the thiourea compound contains a compound represented by the following formula (1): 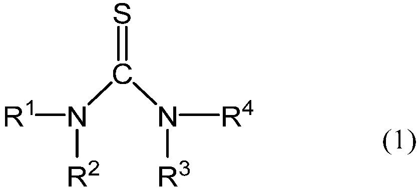
in the formula (1), R¹ to R⁴ each independently represent a hydrogen atom, an alkyl group having 1 to 4 carbon atoms, an allyl group, an acetyl group, or a group represented by the following formula (2): 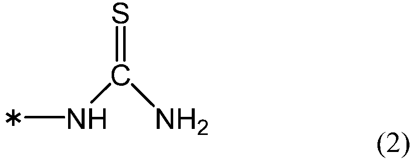
in the formula (2), * represents a bonding position in the compound represented by the formula (1).

3. The test reagent according to claim 1 or 2, wherein a content of the thiourea compound in the test reagent is 0.0005 M or more and 2 M or less.

4. The test reagent according to any one of claims 1 to 3, wherein the thiourea compound contains thiourea.

5. The test reagent according to any one of claims 1 to 4, further comprising at least one kind of buffer selected from the group consisting of: MES; Bis-Tris; ADA; PIPES; ACES; BES; MOPSO; MOPS; TES; HEPES; TAPSO; POPSO; HEPSO; EPPS; Tricine; Bicine; TAPS; CHES; and CAPS.

6. The test reagent according to any one of claims 1 to 5, further comprising a chelating agent.

7. The test reagent according to any one of claims 1 to 6, wherein an amount of the sulfur element in the resin particles determined with an X-ray photoelectron spectrometer is 0.1 atm% or more and 8.0 atm% or less.

8. The test reagent according to any one of claims 1 to 7, wherein an amount of the sulfur element in the resin particles determined with an X-ray photoelectron spectrometer is 0.5 atm% or more and 6.0 atm% or less.

9. The test reagent according to any one of claims 1 to 8, wherein the resin particles each have one of an antibody or an antigen.

10. The test reagent according to any one of claims 1 to 9, wherein the resin particles each have a polymer having, in a main chain thereof, a structure derived from at least one of styrenes and (meth)acrylates.

11. The test reagent according to any one of claims 1 to 10, wherein the resin particles each have a polymer having at least one of a sulfide group and a thiol group.

12. The test reagent according to any one of claims 1 to 11, wherein the resin particles each have a polymer having a hydroxy group.

13. The test reagent according to any one of claims 1 to 12, wherein the resin particles each have a polymer having at least one of a carboxy group and a carboxy group-derived structure.

14. The test reagent according to any one of claims 1 to 13, wherein when a content of the thiourea compound in the test reagent is represented by W(A), and a content of the resin particles in the test reagent is represented by W(B), a mass ratio W(A)/W(B) is 0.2 or more and 450 or less.

15. The test reagent according to any one of claims 1 to 14, wherein the test reagent is a test reagent including two or more liquids including a buffer solution and a resin particle dispersion, the buffer solution is free of the resin particles and contains the thiourea compound, and the resin particle dispersion contains the resin particles.

16. The test reagent according to claim 15, wherein the buffer solution has a pH of 6.0 or more and 9.0 or less.

17. The test reagent according to any one of claims 1 to 16, wherein the test reagent is used in a test by a latex agglutination method.

18. A method of detecting a target substance in a specimen by in vitro diagnosis, the method comprising mixing the test reagent of any one of claims 1 to 17 and a specimen that may contain a target substance.
